# EUROPÄISCHE PATENTSCHRIFT

(11) **EP 1 886 674 B1**
(45) Veröffentlichungstag und Bekanntmachung des Hinweises auf die Patenterteilung: **31.03.2010**
(21) Anmeldenummer: 07113605.5
(22) Anmeldetag: 01.08.2007
(51) Int. Cl.: A61K 9/20, A61K 31/353

(54) **Pharmazeutische Zusammensetzung enthaltend Nebivolol**
Pharmaceutical composition comprising nebivolol
Composition pharmaceutique comprenant du nebivolol

(30) Priorität: 04.08.2006 DE 102006036579; 04.08.2006 US 835606 P
(43) Veröffentlichungstag der Anmeldung: 13.02.2008
(73) Patentinhaber: Alfred E. Tiefenbacher GmbH & Co. KG, 22767 Hamburg (DE)
(72) Erfinder: Pasahn, Manohar, 400 602, THANE WEST (IN); Chary, Bala Ramesha, 500 072, HYDERABAD (IN); Jagadeesh, Thipperudraiah, 577 511, CHITRADURGA (DIST) (IN); Lakshmi, Akkineni, 500 050, Hyderabad (IN); Gnanendra, Thirupathi, 502 032, HYDERABAD (IN)
(74) Vertreter: Feldmann, Ute

(56) Entgegenhaltungen:
- WO-A-95/22325
- WO-A-2006/025070
- WO-A-2006/084684

## Beschreibung

Die vorliegende Erfindung betrifft eine pharmazeutische Zusammensetzung, die als Wirkstoff Nebivolol oder ein pharmazeutisch annehmbares Salz davon in einer Trägerpolymermatrix enthält, sowie ein Verfahren zur Herstellung der Zusammensetzung.

Nebivolol ist die generische Bezeichnung für α,α'-[Iminobis[(methylen)bis-(6-fluorchroman-2-methanol)]. Die Strukturformel von Nebivolol ist im Folgenden gezeigt.

Nebivolol ist eine racemische Mischung zweier Enantiomere mit RSSS- und SRRR-Konfiguration. Das RSSS-Enantiomer wird auch als RS₃-Nebivolol (L-Nebivolol) bezeichnet und das SRRR-Enantiomer wird auch als SR₃-Nebivolol (D-Nebivolol) bezeichnet. Nebivolol ist ein β-Rezeptorenblocker mit hoher β₁-Selektivität bzw. Cardioselektivität, wobei diese Selektivität in erster Linie auf das D-Enantiomer zurückzuführen ist. Zusätzlich besitzt das D-Nebivolol eine über das L-Arginin/Stickoxid-Stoffwechselsystem vermittelte, milde vasodilatierende Eigenschaft, die zur Therapie der essenziellen Hypertonie beiträgt. Aufgrund seines Wirkmechanismus wird Nebivolol zur Behandlung von Hypertonie verwendet.

2,2'-Iminobisethanolderivate werden allgemein in EP 145067 beschrieben,. Sie sind zur Behandlung oder Prävention von Störungen des kardiovaskulären Systems geeignet. [Iminobismethylen]bis-[3,4-dihydro-2H-1-benzopyran-2-methanol]derivate, wie z.B. Nebivolol, werden in EP 334429 beschrieben.

Auch Verfahren zur Herstellung von Nebivolol sind bekannt und werden sowohl in EP 145067 als auch in EP 334429 beschrieben. Nebivolol kann durch Behandlung mit geeigneten Säuren in eine pharmazeutisch annehmbare Salzform überführt werden. Geeignete Säuren hierfür sind z.B. anorganische Säuren, wie beispielsweise HCl, HBr, H₂SO₄, H₃PO₄ oder auch organische Säuren, wie beispielsweise Essigsäure, Malonsäure, Fumarsäure etc. Bevorzugt wird das Hydrochloridsalz des Nebivolols eingesetzt.

Generell bevorzugt sind orale Dosierungsformen, wie z.B. Tabletten, die von Patienten einfach eingenommen werden können. Der therapeutisch wirksame Bestandteil einer oralen Dosierungsform muss dabei in ausreichender Konzentration und über einen bestimmten Zeitraum am Wirkort vorliegen, um die gewünschte Wirkung im Organismus zu erzielen. Das Ausmaß und die Geschwindigkeit, mit der der Wirkstoff aus der pharmazeutischen Zusammensetzung am Wirkort verfügbar ist, wird als Bioverfügbarkeit bezeichnet. Eine entscheidende Voraussetzung für die Bereitstellung einer geeigneten oralen Darreichungsform ist daher eine ausreichend hohe Bioverfügbarkeit des Wirkstoffs. Die Bioverfügbarkeit wird u.a. durch die Geschwindigkeit der Freisetzung und durch die Auflösung des Wirkstoffs bestimmt. Der erste die Verfügbarkeit am Wirkort bestimmende Schritt nach Applikation einer Arzneiform ist die Freigabe aus der Arzneiform. Bei festen Arzneiformen, wie z.B. der Tablette, setzt sich die Freigabe aus dem Zerfall der Tablette in kleine Partikel, auch als Desintegration bezeichnet, und der Auflösung des Wirkstoffs in der am Resorptionsort vorliegenden Flüssigkeit zusammen. Der gelöste Wirkstoff kann dann über den Gastrointestinaltrakt resorbiert und gegebenenfalls zum Wirkungsort transportiert werden.

Orale Dosierungsformen durchwandern zuerst den Magen mit dem sauren Magensaftmilieu und kommen dann in den Darmtrakt mit einem etwas alkalischeren Milieu. Die Zeit, die zur Auflösung und Resorption zur Verfügung steht, ist daher begrenzt. Wenn beim Durchwandern des Arzneistoffes durch die Resorptionszone des Magen-Darm-Trakts keine vollständige Auflösung und Resorption erzielt wird, kann eine ausreichend hohe und therapeutisch wirksame Wirkstoffkonzentration im Plasma nicht erreicht werden. Die Auflösungsfähigkeit des Wirkstoffs ist daher ein sehr wesentlicher Faktor. Die Formulierung einer geeigneten oralen Arzneimittelform, wie z.B. einer Tablette, stellt insbesondere bei schwer löslichen Wirkstoffen eine besondere Herausforderung dar und ist aufgrund der unterschiedlichsten Auflösecharakteristiken und des Resorptionsverhaltens eines jeden einzelnen Wirkstoffs jeweils individuell zu entwickeln und anzupassen, um die geforderte Bioverfügbarkeit zu erreichen. Neben der Bioverfügbarkeit muss bei der Entwicklung einer Tablette darüber hinaus den Erfordernissen einer später im industriellen Maßstab durchgeführten Massenproduktion mit den dazu notwendigen Qualitätskontrollen Rechnung getragen werden.

Wie in EP 744946 ausgeführt, zählt Nebivolol-Hydrochlorid zu den schwer löslichen Wirkstoffen, und zwar sowohl in normaler kristalliner Form als auch in mikronisierter Form. Die in der pharmazeutischen Technologie zur Erhöhung der Bioverfügbarkeit eines Wirkstoffs angewandte Standardtechnik der Mikronisierung versagt somit beim Nebivolol-Hydrochlorid.

In EP 744946 wird das Problem dadurch gelöst, dass das Nebivolol-Hydrochlorid mit einem Netzmittel kombiniert wird, wobei das Verhältnis von mikronisiertem Wirkstoff zu Netzmittel in einem Bereich von 0,025 bis 0,5 liegen soll. In EP 744946 wurde festgestellt, dass Polysorbat 80 hierzu wirkungsvoll eingesetzt werden kann. In der Literatur wurde allerdings gefunden, dass Polysorbat 80 eine allergene Wirkung haben kann und anaphylaktische Reaktionen auslösen kann.

E.A. Coors et al. beschreiben in "Annals of Allergy, Asthma and Immunolgy", Bd. 95, Nr. 6, Dezember 2005, Seiten 593-599 (7) "Polysorbate 80 in medical products and non-immunologic anaphylactoid reactions", dass Polysorbat 80 ein "verdeckter" Induktor von anaphylaktischen Reaktionen ist und schwere anaphylaktische Reaktionen nicht immunologischen Ursprungs bei Patienten verursacht hat.

Tabletten mit Nebivolol werden in WO 2006/025070 A offenbart.

Es war daher Aufgabe der Erfindung, eine Zusammensetzung, die Nebivolol oder ein pharmazeutisch annehmbares Salz davon als Wirkstoff enthält, in einer solchen Form bereitzustellen, die eine ausreichende Löslichkeit hat, um während des Durchwanderns des Magen-Darm-Trakts resorbiert zu werden, ohne ein Netzmittel, insbesondere Polysorbat 80 verwenden zu müssen.

Diese Aufgabe wird gelöst mit pharmazeutischen Zusammensetzungen, Verfahren zu deren Herstellung sowie Verwendungen von Nebivolol, wie sie in Ansprüchen 15 und 16 beschrieben werden.

Die erfindungsgemäße Zusammensetzung ist frei bzw. im Wesentlichen frei von Netzmitteln, wobei unter "im Wesentlichen frei" verstanden wird, dass die Zusammensetzung nur allenfalls eingeschleppte unvermeidliche Anteile von Netzmitteln enthält. Es wird jedoch kein Netzmittel zugesetzt, um die Löslichkeit des Wirkstoffs zu erhöhen.

Der Wirkstoff ist Nebivolol oder ein pharmazeutisch annehmbares Salz davon. Pharmazeutisch annehmbare Salze für Wirkstoffe wie Nebivolol sind bekannt und die üblichen sind hier anwendbar. Üblicherweise wird als pharmazeutisch annehmbares Salz das Salz einer anorganischen oder organischen Säure verwendet. Üblich sind Hydrochloride, Hydrobromide, Sulfate, Phosphate sowie Acetate, Malonate, Fumarate etc. Bevorzugt wird das Hydrochlorid in Betracht gezogen. Besonders bevorzugt ist der Wirkstoff in der erfindungsgemäßen Zusammensetzung daher Nebivolol-Hydrochlorid.

Der Wirkstoff in der erfindungsgemäßen Zusammensetzung liegt in mikronisierter Form vor. Unter mikronisierter Form wird ein Wirkstoff verstanden, dessen Teilchengröße im Mikrometer- oder Nanometerbereich liegt. In Betracht kommen unter anderem solche Wirkstoffe, deren volumenmittlerer Teilchendurchmesser im Bereich von 0,01 bis 100 µm, bevorzugt 0,5 bis 50 µm und besonders bevorzugt 1 bis 20 µm liegt, wobei die Teilchengrößenbestimmung mit einem Malvern Mastersizer unter Verwendung von Laserlichtstreuung in an sich bekannter Weise erfolgen kann. Von den Teilchen haben bevorzugt mehr als 80% einen Durchmesser von < 50 µm und besonders bevorzugt mehr als 90%.

In einer bevorzugten Ausführungsform werden mikronisierte Teilchen mit einer spezifischen Oberfläche von mehr als 1,8 m²/g, bevorzugt mehr als 2,5 m²/g, besonders bevorzugt mehr als 2,8 m²/g und insbesondere mehr als 3,0 m²/g verwendet.

Zur Mikronisierung des Wirkstoffs können die an sich bekannten üblichen Verfahren eingesetzt werden, z.B. Mahlung in dafür geeigneten Mühlen, wie z.B. Luftstrahl- oder Kugelmühlen, Ausfällung aus Lösemitteln, Sprühtrocknung, Siebung oder auch neuere Techniken, wie z.B. Ausfällung aus überkritischen Fluiden, wie z.B. Kohlendioxid, gegebenenfalls in Kombination mit organischen Lösemitteln.

Da der Wirkstoff auch in mikronisierter Form nicht solche Auflösungseigenschaften aufweist, dass er sich in ausreichender Weise im Magen-Darm-Trakt auflöst und resorbiert werden kann, wird erfindungsgemäß eine Matrix aus einem Polymerträgermaterial umfassend oder bestehend aus Polyvinylpyrollidon-co-vinylacetat bereitgestellt, die für die bessere Auflösbarkeit sorgt. Überraschenderweise wurde festgestellt, dass dann, wenn der Wirkstoff in Form einer Dispersion in einer Lösung das Polymerträgermaterials verarbeitet wird, seine Löslichkeitseigenschaften so stark verbessert werden, dass der Einsatz in Tablettenform kein Problem mehr darstellt, wobei jedoch auf die Verwendung von Benetzungsmitteln verzichtet werden kann.

Es wurde gefunden, dass die Löslichkeitseigenschaften mit einem Polymermaterial umfassend oder bestehend aus Polyvinylpyrollidon-co-vinylacetat, das der Zusammensetzung Struktur verleihen kann und z.B. eine Matrix bilden kann, stark verbessert werden. Ein solches Polymermaterial wird gemeinsam mit dem Wirkstoff eingesetzt und umschließt und stabilisiert diesen. Geeignet sind hierzu Polymere umfassend oder bestehend aus Polyvinylpyrollidon-co-vinylacetat, die sich leicht in Wasser oder wässrigem Milieu lösen und in entwässertem oder getrocknetem Zustand eine amorphe Matrix bilden, innerhalb der die Wirkstoffteilchen eingelagert werden können. Ohne an eine Theorie gebunden zu sein, wird davon ausgegangen, dass durch die Art der Verarbeitung die Wirkstoffteilchen durch die Polymerträgermatrix umfassend oder bestehend aus Polyvinylpyrollidon-co-vinylacetat getrennt bleiben, selbst wenn die Dispersion zusammen mit weiteren Bestandteilen der pharmazeutischen Zusammensetzung zu Tabletten verarbeitet wird. Aus dieser Matrix wird der Wirkstoff dann, wenn er in das Milieu des Magensaftes gelangt, schnell freigesetzt und gleichzeitig gelöst, so dass er im Darm dann zur Resorption zur Verfügung steht. Auf diese Art und Weise werden sehr vorteilhafte Resorptionseigenschaften erzielt.

Als Trägermaterial sind solche Polymere umfassend oder bestehend aus Polyvinylpyrollidon-co-vinylacetat geeignet, die eine inerte, wasserlösliche Matrix bilden, die den Wirkstoff aufnehmen können. Hier können sowohl synthetische als auch natürliche Polymere als auch Derivate von natürlichen Polymeren genannt werden. Insbesondere kommen in Betracht Cellulosederivate und Pyrrolidonpolymere und -Copolymere. Als besonders geeignet haben sich Polyvinylpyrrolidon, als Povidone im Handel, Polyvinylpyrrolidon-co-vinylacetat, u.a. als Copovidone im Handel erhältlich, Hydroxypropylcellulose, Hydroxypropylmethylcellulose, Hydroxypropylmethylcellulosephthalat und Polyethylenglycol erwiesen. Als Polyvinylpyrrolidon eignet sich z.B. eines mit einem Molekulargewicht im Bereich von 30.000 bis 100.000, besonders bevorzugt im Bereich von 50.000.

Es hat sich gezeigt, dass für optimale Abgabeeigenschaften das Verhältnis von Polymermatrix zu Wirkstoff ein wichtiger Parameter ist. Wenn das Verhältnis zu sehr auf Seiten des Wirkstoffs liegt, reicht die Menge des Polymers nicht aus, um die Teilchen voneinander zu trennen oder an der Aggregation zu hindern. Ist das Verhältnis zu stark auf die Seite des Polymers verschoben, ist insgesamt der Anteil an Wirkstoff und Polymer in der Zusammensetzung zu hoch, um gut verarbeitet werden zu können.

Als geeignet hat sich ein Verhältnis von Wirkstoff zu Polymerträgermaterial im Bereich von 1:0,8 bis 1:10 erwiesen, bevorzugt im Bereich von 1:1 bis 1:10. Weiter bevorzugt liegt das Verhältnis in einem Bereich von 1:1 bis 1:6.

Die erfindungsgemäße Formulierung der vorliegenden Erfindung kann als zusätzliche Bestandteile pharmazeutisch annehmbare Trägermittel und Hilfsstoffe enthalten, wie Füllstoffe, Schmiermittel, Zerfallhilfsmittel, und Bindemittel. Beispiele für geeignete Füllstoffe sind Lactose, mikrokristalline Cellulose oder Calciumhydrogenphosphat, Schmiermittel, wie Stearinsäure, Magnesiumstearat, Talkum oder Silica; Sprengmittel, wie Stärke unterschiedlicher Herkunft, Stärkederivate, Natriumstärkeglycolat, chemisch vernetzte Polymere oder Copolymere, wie vernetztes Polyvinylpyrrolidon oder vernetzte Carboxymethylcellulose, die auch unter dem Namen Croscarmellose oder Croscarmellose-Natrium im Handel erhältlich ist; Bindemittel, wie Maisstärke.

Auch Aromastoffe können in der erfindungsgemäßen Zusammensetzung enthalten sein.

Eine erfindungsgemäße pharmazeutische Zusammensetzung, die als Wirkstoff Nebivolol oder ein Salz davon in mikronisierter Form in einer Polymerträgermatrix umfassend oder bestehend aus Polyvinylpyrollidon-co-vinylacetat zusammen mit bekannten, handelsüblichen pharmazeutischen Hilfsmitteln enthält, kann mit Standardtechniken hergestellt werden und führt zu einer ausreichend hohen Bioverfügbarkeit. Gleichzeitig genügt sie den Erfordernissen an eine Fertigung im industriellen Maßstab. Erfindungsgemäß wird somit eine besonders vorteilhafte feste Formulierung für Nebivolol bereitgestellt, die gleichzeitig ein ausgezeichnetes Löslichkeitsverhalten besitzt.

Der erfindungsgemäße Wirkstoff wird mit bekannter Technik auf eine Teilchengröße im Mikrometer- oder Nanometerbereich zerkleinert. In dieser Form kann er dann mit dem in Wasser oder wässrigem Medium gelösten Polymer umfassend oder bestehend aus Polyvinylpyrollidon-co-vinylacetat dispergiert und auf diese Weise verarbeitet werden. Auf diese Weise wird der Wirkstoff fein verteilt. Die Polymerträgermatrix umfassend oder bestehend aus Polyvinylpyrollidon-co-vinylacetat wird in Wasser oder einem wässrigen Medium gelöst. Als wässriges Medium kommen die üblicherweise zur Herstellung von pharmazeutischen Formulierungen eingesetzten Medien in betracht. In der Regel wird Wasser, in destillierter oder sonstiger reiner Form, verwendet, aber auch eine Mischung aus Wasser und einem Alkohol oder anderem Lösungsmittel ist geeignet.

Die erfindungsgemäße Zusammensetzung muss so zubereitet werden, dass bei der Herstellung das Trägerpolymer umfassend oder bestehend aus Polyvinylpyrollidon-co-vinylacetat eine Matrix oder Struktur bilden kann, in die der Wirkstoff eingelagert wird oder von der er umschlossen ist. Das Matrixmaterial muss wasserlöslich sein, um eine wässrige Dispersion bilden zu können. Diese wässrige Dispersion kann dann für die Nassgranulierung verwendet werden. Dadurch kann der Wirkstoff in der Lösung des Trägermaterials sehr einfach verteilt werden. Durch die anschließende Stufe der Nassgranulierung wird die Matrix ausgebildet, die dann den Wirkstoff umschließt. Die Matrix das Strukturmittel schützt den Wirkstoff während der nachfolgenden Stufen und verhindert eine Aggregierung des Wirkstoffs, was wieder schwer löslichere Produkte ergäbe, und schützt auch mechanisch bei der Tablettenherstellung. Auf diese Art und Weise entsteht ein sehr gut lösliches Produkt, von dem in einer nachgeahmten Magensaftlösung nach 1 Stunde mindestens 80%, bevorzugt mindestens 90% gelöst sind.

Die erfindungsgemäße Zubereitung liegt bevorzugt in einer oralen Dosierungsform vor. Als orale Dosierungsformen sind Tabletten, Kapseln, Granulate, und Pastillen und andere Formen geeignet. Bevorzugt liegt die erfindungsgemäße Zusammensetzung in Form von Tabletten vor.

Die Erfindung wird noch durch die folgenden Beispiele erläutert, die jedoch keinesfalls als beschränkend ausgelegt werden sollten.

### Beispiel 1

Es wurden zwei Tablettenformulierungen hergestellt mit den folgenden Komponenten Beispiel 1(1) ist nicht erfindungsgemäß.

| Komponenten | Beispiel 1 | Beispiel 2 |
|---|---|---|
| **Wirkstoffdispersion** | | |
| Nebivolol-Hydrochlorid | 5,45 | 5,45 |
| Povidone K30 | 24,55 | -- |
| Copovidone | -- | 20,00 |
| reines Wasser | q.s. | q.s. |

| **Hilfsstoffe zur Granulierung** | | |
|---|---|---|
| Lactose-Monohydrat | 127,00 | 141,55 |
| Cellulose, mikrokristallin | 60,00 | 50,00 |
| Crospovidone | 6,00 | 6,00 |

| **Weitere Zusatzstoffe, Schmiermittel** | | |
|---|---|---|
| Crospovidone | 6,00 | 6,00 |
| Magnesiumstearat | 1,00 | 1,00 |

### Herstellungsverfahren:

Lactose-Monohydrat, mikrokristalline Cellulose und Crospovidone wurden in einem Hochleistungsmischer vermischt. Getrennt davon wurde Povidone bzw. Copovicone in Wasser gelöst. In der Polymerlösung wurde das Nebivolol-Hydrochlorid dispergiert. Die Wirkstoff/Polymer-Dispersion wurde dann der Lactosemischung zugefügt und die Masse einer Nassgranulierung unterzogen. Die Körnchen wurden in einem FBD getrocknet, gesiebt und mit Crospovidone und Magnesiumstearat vermischt.

Die vermischten Körnchen wurden zu bikonvexen Tabletten mit 8,5 mm Durchmesser gepresst.

Es wurden Tabletten mit ausgezeichneten Eigenschaften erhalten.

### Beispiel 2

Zur Herstellung von Tabletten wurden die folgenden Inhaltsstoffe eingesetzt:

| | Inhaltsstoffe | NEB/A-030 (Basierend auf NEB/A-003) mg/Tablette |
|---|---|---|
| **Stufe A (Trockene Mischung)** | | |
| 1 | Lactose-Monohydrat | 141,550 |
| 2 | Cellulose, mikrokristallin | 50,000 |
| 3 | Crospovidone | 6,000 |
| 4 | Maisstärke | |

| **Stufe B (Feste Dispersion, Herstellung und Granulierung)** | | |
|---|---|---|
| 1 | Nebivolol-Hydrochlorid | 5,450 |
| 2 | Plasdone S-630 | 5,000 |
| 3 | Reines Wasser mit 70°C | q.s. |
| 4 | Reines Wasser | q.s. |

| **Stufe C (Mischen u. Gleitmittel)** | | |
|---|---|---|
| 1 | Magnesiumstearat | 1,000 |
| 2 | Crospovidone | 6,000 |
| **Gesamttablettengewicht** | | 215,00 |

Aus diesen Komponenten wurde mit dem Verfahren von Beispiel 1 eine Tablettenformulierung hergestellt. Die erhaltenen Tabletten lösten sich in Magensaft-Imitat gut auf.

### Beispiel 3

| | Inhaltsstoffe | NEB/A-026 mg/Tablette | NEB/A-030 (Basierend auf NEB/A-003) mg/Tablette |
|---|---|---|---|
| **Stufe A (Trockene Mischung)** | | | |
| 1 | Lactose-Monohydrat | 158,00 | 141,550 |
| 2 | Cellulose, mikrokristallin | -- | 50,000 |
| 3 | Crospovidone | -- | 6,00 |
| 4 | Maisstärke | 30,00 | |

| **Stufe B (Feste Dispersion, Herstellung und Granulierung)** | | | |
|---|---|---|---|
| 1 | Nebivolol-Hydrochlorid | 5,5 | 5,450 |
| 2 | Plasdone S-630 | 5,0 | 5,000 |
| 3 | Reines Wasser mit 70°C | q.s. | q.s. |
| 4 | Reines Wasser | q.s. | q.s. |

| **Stufe C (Mischen u. Gleitmittel)** | | | |
|---|---|---|---|
| 1 | Magnesiumstearat | 1,50 | 1,000 |
| 2 | Crospovidone | -- | 6,000 |
| **Gesamttablettengewicht** | | 200,00 | 215,00 |

| | | | |
|---|---|---|---|
| Teilchengröße D_{0,9} = 13,2 µm. | | | |

Aus den oben angegebenen Komponenten wurden, wie in Beispiel 1 beschrieben, Tabletten hergestellt. Von diesen und zum Vergleich von einem Handelsprodukt, Nebilet, wurde ein in-vitro-Auflösungsprofil erstellt. Hierzu wurden jeweils einige Tabletten in ein Gefäß mit einem Paddelrührer gegeben, wobei der Paddelrührer mit einer Geschwindigkeit von 50 U/min betrieben wurde. Das Volumen des Gefäßes war 900 ml. Als Medium wurde 0,1 n Salzsäure verwendet, um die Magensäure nachzuahmen. Nach 5, 10, 15, 30, 45 und 60 Minuten wurde bestimmt, wie viel von dem Produkt sich gelöst hatte. Die folgenden Ergebnisse wurden erhalten.

| Zeit | Referenzprodukt | | NEB/A-030 |
|---|---|---|---|
| 5 min | 42 | 32 | 61 |
| 10 min | 71 | 78 | 75 |
| 15 min | 83 | 86 | 79 |
| 30 min | 93 | 87 | 83 |

### Beispiel 4

Für eine erfindungsgemäße Zusammensetzung wurden physikalische Parameter und Auflösungsdaten bestimmt: Aus folgenden Bestandteilen wurden Tabletten hergestellt, wie in Beispiel 1 beschrieben: Zum Vergleich wurden Nebilet-Tabletten auf gleiche Art und Weise untersucht. Die Ergebnisse sind unten angegeben. NEB/A-001 ist nicht erfindungsgemäß.

| Inhaltsstoffe | NEB/A-001 | NEB/A-002 |
|---|---|---|
| **Wirkstoffdispersion:** | | |
| Nebivolol-Hydrochlorid | 5,45 | 5,45 |
| Povidone K30 | 24,55 | -- |
| Copovidone | -- | 20,00 |
| Reines Wasser | q.s. | q.s. |

| **Hilfsstoffe zur Granulierung:** | | |
|---|---|---|
| Lactose-Monohydrat | 127,00 | 141,55 |
| Cellulose, mikrokristallin | 60,00 | 50,00 |
| Crospovidone | 6,00 | 6,00 |

| **Hilfsstoffe u. Schmiermittel** | | |
|---|---|---|
| Crospovidone | 6,00 | 6,00 |
| Magnesiumstearat | 1,00 | 1,00 |
| **Tablettengewicht** | 230,00 | 230,00 |

| **Physikalische Parameter** | | | | |
|---|---|---|---|---|
| **Parameter** | **Nebilet^{®}** (Lot 44578) | **Nebilet^{®}** (Lot 51503) | **NEB/A-001** | **NEB/A-002** |
| LOD d. Körnchen (% m/m) | -- | -- | 1,79 | 1,27 |
| Durchschnittsgew. (mg) | 230 | 230 | 230 | 230 |
| Dicke (mm) | 3,08 | 3,07 | 3,20 | 2,87 |
| Härte (Newton) | 87 | 98 | 93 | 95 |
| Zeit bis zum Zerfall | 2 min 27 s | 2 min 03 s | 2 min 50 s | 2 min 05 s |

| **Auflösungsprofil (Kumulativ % Wirkstoff freigesetzt):** | | | | |
|---|---|---|---|---|
| Medium: 0,1 n Salzsäure (900 ml); Vorrichtung: Paddelrührer - 50 U/min | | | | |

| **Zeit (min)** | **Nebilet®** (Lot 44578) | **Nebilet®** (Lot 51503) | **NEB/A-001** | **NEB/A-002** |
|---|---|---|---|---|
| 5 | 40 | 44 | 67 | 67 |
| 15 | 78 | 88 | 93 | 104 |
| 30 | 88 | 103 | 94 | 102 |
| 45 | 87 | 103 | 93 | 101 |
| 60 | 87 | 104 | 92 | 102 |

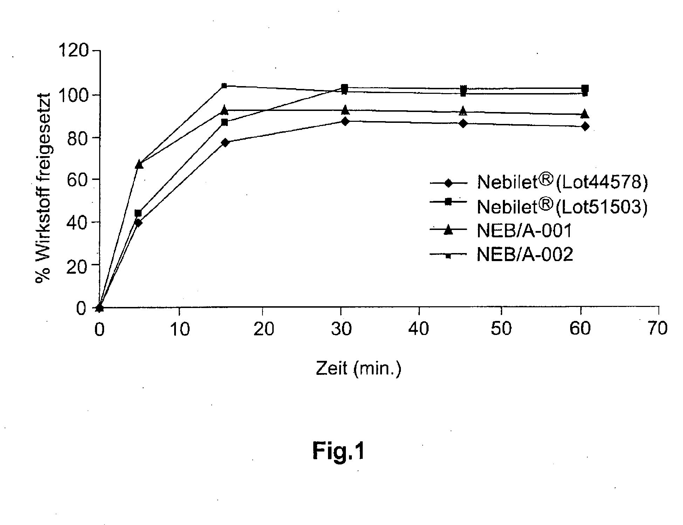

Ein Diagramm, das das Auflösungsprofil zeigt, ist in Figur 1 zu sehen. Es wird das Profil von bekannten Nebivolol-Tabletten im Vergleich zu erfindungsgemäßen Tabletten, mit jeweils 5 mg Wirkstoff, gezeigt.

### Beispiel 5

Aus den folgenden Inhaltsstoffen wurden Tabletten hergestellt.

| Inhaltsstoffe | Milligramm pro Tablette |
|---|---|
| Nebivolol-Hydrochlorid | 5,45 |
| Copovidone | 5,00 bis 15,00 |
| Lactose-Monohydrat | 156,45 bis 146,55 |
| Cellulose, mikrokristallin | 50,00 |
| Crospovidone | 12,00 |
| Magnesiumstearat | 1,00 |
| Tablettengewicht | 230,00 |

### Beispiel 6

Um die optimale Menge an Copovidone festzustellen, wurden sechs Zusammensetzungen hergestellt und die physikalischen Parameter getestet. Die Zusammensetzung der einzelnen Formulierungen und die Ergebnisse der Tests sind in der folgenden Tabelle angegeben.

| Inhaltsstoffe | F.1 | F.2 | F.3 | F.4 | F.5 | F. 6 |
|---|---|---|---|---|---|---|
| **Stufe A (Trockene Mischung)** | | | | | | |
| Lactose-Monohydrat Ph. Eur. | 127,00 | 141,55 | 141,55 | 141,55 | 141,55 | 141,55 |
| Cellulose, mikrokristallin Ph. Eur. | 60,00 | 50,0 | 50,00 | 50,00 | 50,00 | 40,00 |
| Crospovidone Ph. Eur. | -- | 6,00 | 6,00 | 6,00 | 6,00 | 6,00 |

| **Stufe B (Feste Dispersion u. Granulierung)** | | | | | | |
|---|---|---|---|---|---|---|
| Nebivolol-Hydrochlorid | 5,45 | 5,45 | 5,45 | 5,45 | 5,45 | 5,45 |
| Povidone K30 | 24,55 | -- | -- | -- | -- | -- |
| Copovidone Ph. Eur. | -- | 5,00 | 10,00 | 15,00 | 20,00 | 5,00 |
| Wasser, ger. Ph. Eur. (60°-70°C) | q.s. | q.s. | q.s. | q.s. | q.s. | q.s. |
| Wasser, ger. Ph. Eur. | q.s. | q.s. | q.s. | q.s. | q.s. | q.s. |

| **Stufe C (Vermischen)** | | | | | | |
|---|---|---|---|---|---|---|
| Crospovidone Ph. Eur. | 6,00 | 6,00 | 6,00 | 6,00 | 6,00 | -- |
| Magnesiumstearat Ph. Eur. | 1,0 | 1,00 | 1,00 | 1,0 | 1,0 | 1,00 |
| Tablettengewicht | 230 | 215 | 220 | 225 | 230 | 200 |
| Härte (Newton) | 93 | 98 | 99 | 100 | 95 | 97 |
| Brüchigkeit | Nil | Nil | Nil | Nil | Nil | Nil |
| Zeit bis zum Zerfall | 3 min | 1 min | 2 min | 1 min | 2 min | 1 min |

| Auflösung 900 ml, 0,1 n HCl, Paddelrührer 50 U/min | | | | | | |
|---|---|---|---|---|---|---|
| 5 min | 69 | 70 | 57 | 72 | 67 | 62 |
| 15 min | 93 | 87 | 80 | 89 | 104 | 90 |
| 30 min | 94 | 91 | 80 | 90 | 102 | 94 |
| 45 min | 93 | 92 | 81 | 91 | 101 | 93 |
| 60 min | 92 | 93 | 83 | 91 | 102 | 93 |

Alle Chargen wurden in befriedigender Weise hergestellt mit vergleichbarem Auflösungsprofil, für die weiteren Versuche wurden 5 mg Copovidone ausgewählt. Formulierung F.1 ist nicht erfindungsgemäß.

### Beispiel 7

**Optimierung der Menge an Verdünnungsmittel**

| Inhaltsstoffe | F.7 | F.8 | F.9 | F.10 | F.11 | F.12 |
|---|---|---|---|---|---|---|
| **Stufe A (Trockene Mischung)** | | | | | | |
| Lactose-Monohydrat Ph. Eur. | 148,05 | 148,05 | 158,05 | 158,05 | 199,65 | 168,55 |
| Cellulose, mikrokristallin Ph. Eur. | -- | 10,00 | -- | -- | -- | 20,00 |
| Maisstärke | 40,000 | 30,00 | 30,00 | 30,00 | 38,00 | -- |
| Crospovidone Ph. Eur. | -- | -- | -- | -- | -- | 4,00 |

| **Stufe B (Feste Dispersion u. Granulierung)** | | | | | | |
|---|---|---|---|---|---|---|
| Nebivolol-Hydrochlorid | 5,45 | 5,45 | 5,45 | 5,45 | 5,45 | 5,45 |
| Copovidone Ph. Eur. | 5,00 | 5,00 | 5,00 | 5,00 | 5,00 | 5,00 |
| Wasser, ger. Ph. Eur. | q.s. | q.s. | q.s. | q.s. | q.s. | q.s. |

| **Stufe C (Vermischen)** | | | | | | |
|---|---|---|---|---|---|---|
| Magnesiumstearat Ph. Eur. | 1,50 | 1,50 | 1,50 | 2,00 | 1,90 | 1,50 |
| Tablettengewicht | 200 | 200 | 200 | 200 | 250 | 200 |
| Härte (Newton) | 101 | 96 | 90 | 82 | 104 | 90 |
| Brüchigkeit | Nil | Nil | Nil | Nil | Nil | Nil |
| Zeit bis zum Zerfall | 3 min | 3 min | 3 min | 3 min | 3 min | 1 min |

| Auflösung 900 ml, 0,1 n HCl, Paddelrührer 50 U/min | | | | | | |
|---|---|---|---|---|---|---|
| 5 min | 47 | 53 | 40 | 43 | 50 | 71 |
| 15 min | 73 | 86 | 85 | 83 | 87 | 101 |
| 30 min | 85 | 105 | 98 | 102 | 100 | 102 |
| 45 min | 94 | 103 | 99 | 100 | 99 | 101 |
| 60 min | 95 | 105 | 99 | 101 | 100 | 101 |

### Beispiel 8

Um zu zeigen, dass die erfindungsgemäße Zusammensetzung durch ihre speziell strukturierte Form verbesserte Eigenschaften zeigt, wurden zum Vergleich Tabletten mit im wesentlichen denselben Inhaltsstoffen hergestellt, ohne dass eine Matrixbildung erfolgt. Komponenten und Ergebnisse sind der folgenden Tabelle zu entnehmen.

**Zugabe des Wirkstoffs ohne Dispersion (Vergleichsbeispiel)**

| Inhaltsstoffe | F. 13 | F. 14 | F. 15 | F. 16 |
|---|---|---|---|---|
| **Stufe A (Trockene Mischung)** | | | | |
| Nebivolol-Hydrochlorid | 5,45 | 5,45 | 5,45 | 5,45 |
| Lactose-Monohydrat Ph. Eur. | 142,55 | 158,05 | 158,05 | 163,05 |
| Cellulose, mikrokristallin Ph. Eur. | 40,00 | -- | -- | |
| Crospovidone Ph. Eur. | 6,00 | -- | -- | |
| Maisstärke Ph. Eur. | -- | 30,00 | 30,00 | 30,00 |
| Copovidone Ph. Eur. | -- | | 5,00 | -- |

| **Stufe B (Feste Dispersion u. Granulierung)** | | | | |
|---|---|---|---|---|
| Copovidone Ph. Eur. | 5,00 | 5,00 | -- | |
| Wasser, ger. Ph. Eur. | q.s. | q.s. | q.s. | q.s. |

| **Stufe C (Vermischen)** | | | | |
|---|---|---|---|---|
| Crospovidone Ph. Eur. | -- | -- | -- | -- |
| Magnesiumstearat Ph. Eur. | 1,00 | 1,50 | 1,50 | 1,50 |
| Tablettengewicht | 200 | 200 | 200 | 200 |
| Härte (Newton) | 98 | 75-90 | 75-90 | 75-90 |
| Brüchigkeit | Nein | Nein | Nein | Nein |
| Zeit bis zum Zerfall | 5 min | 2 min | 2 min | 1 min |

| Auflösung 900 ml, 0,1 n HCl, Paddelrührer 50 U/min | | | | |
|---|---|---|---|---|
| 5 min | 82 | 58 | 62 | 56 |
| 15 min | 90 | 93 | 94 | 94 |
| 30 min | 91 | 95 | 96 | 94 |
| 45 min | 91 | 95 | 96 | 94 |
| 60 min | 90 | 95 | 96 | 94 |

### Beispiel 9

| Inhaltsstoffe | F.17 | F.18 | F.19 | F.20 |
|---|---|---|---|---|
| **Stufe A (Trockene Mischung)** | | | | |
| Lactose-Monohydrat Ph. Eur. | 141,55 | 141,55 | 141,55 | 141,55 |
| Cellulose, mikrokristallin Ph. Eur. | 50,00 | 50,00 | 50,00 | 50,00 |
| Maisstärke | | | | |
| Crospovidone Ph. Eur. | 6,00 | 6,00 | 6,00 | 6,00 |

| **Stufe B (Feste Dispersion u. Granulierung)** | | | | |
|---|---|---|---|---|
| Nebivolol-Hydrochlorid | 5,45 | 5,45 | 5,45 | 5,45 |
| Copovidone Ph. Eur. | -- | | 5,00 | 5,00 |
| Wasser, ger. Ph. Eur. | q.s. | q.s. | q.s. | q.s. |
| HPC | 5,00 | | | |
| HPMC | | 5,00 | | |

| **Stufe C (Vermischen)** | | | | |
|---|---|---|---|---|
| Crospovidone Ph. Eur. | 1,00 | 1,00 | 1,00 | 1,00 |
| Magnesiumstearat Ph. Eur. | 6,00 | 6,00 | 6,00 | 6,00 |
| Tablettengewicht | 215 | 215 | 215 | 215 |
| Härte (Newton) | 70-90 | 70-90 | 70-90 | 70-90 |
| Brüchigkeit | Nein | Nein | Nein | Nein |
| Zeit bis zum Zerfall | 1 min | 1 min | 1 min | 2 min |

| Auflösung 900 ml, 0,1 n HCl, Paddelrührer 50 U/min | | | | |
|---|---|---|---|---|
| 5 min | 89 | 69 | 57 | 69 |
| 10 min | 100 | 86 | 78 | 84 |
| 15 min | 101 | 92 | 81 | 88 |
| 30 min | 102 | 95 | 90 | 98 |
| 45 min | 103 | 95 | 95 | 98 |
| 60 min | 103 | 97 | 96 | 98 |

Formulierungen F.17 und F.18 sind nicht erfindungsgemäß.

### Beispiel 10 (nicht erfindungsgemäß)

Mit den nicht erfindungsgemäßen Zusammensetzungen (Bsp.11) wurden Tabletten wie folgt hergestellt und mit handelsüblichen Tabletten verglichen:

**Nebivolol-Hydrochlorid-Tabletten mit 5 mg**

| Tablettenparameter: | | |
|---|---|---|
| Parameter | F.Nr.: NEB/A-033 | F.Nr.: NEB/A-034 |
| Durchschnittsgewicht | 215,00 mg | 215,00 mg |
| Zeit bis zum Zerfall | 1 min | 1 min |
| Härte | 60 - 100 N | 60 - 100 N |
| Dicke | 3,1 - 3,3 mm | 3,1 - 3,3 mm |
| Brüchigkeit | Nein | Nein |

### In-vitro-Auflösungsprofil

- Vorrichtung:: Paddelrührer
- Geschwindigkeit:: 50 U/min
- Volumen:: 900 ml
- Medien:: 0,1 n Salzsäure Phosphatpuffer pH 6,8
- Zeitpunkte zur Messung:: 5, 10, 15, 30, 45 und 60 min

Auflösungsprofile von Nebivolol-Hydrochlorid-Tabletten mit 5 mg Wirkstoff im Vergleich

| Medien | Produkt | Mittelwert kumulativ an % Wirkstoff freigesetzt | | | | | |
|---|---|---|---|---|---|---|---|
| | | 5 min | 10 min | 15 min | 30 min | 45 min | 60 min |
| 0,1 n Salzsäure | Nebilet^{®} 5 mg | 42 | 71 | 83 | 93 | 94 | 95 |
| | F.Nr.: NEB/A-033 (Mit HPC) | 89 | 100 | 101 | 102 | 103 | 103 |
| | F.Nr.: NEB/A-034 (Mit HPMC) | 69 | 86 | 92 | 95 | 95 | 97 |
| Phosphat-puffer pH 6,8 | Nebilet^{®} 5 mg | 1,8 | 2,9 | 3,4 | 6,9 | 10,1 | 12,4 |
| | F.Nr.: NEB/A-033 (Mit HPC) | 6 | 6,1 | 7,6 | 14,1 | 19,1 | 25,3 |
| | F.Nr.: NEB/A-034 (Mit HPMC) | 8,2 | 10,1 | 11,7 | 16,5 | 23,3 | 29,7 |

### Beispiel 11 (nicht erfindungsgemäß)

**Nebivolol-Hydrochlorid-Tabletten 5 mg**

| Technik: Dispersionsgranulierung | | | |
|---|---|---|---|
| Zusammensetzung | | | |
| | Inhaltsstoffe | mg/Tablette B.Nr.: NEB/A-033 | mg/Tablette B.Nr.: NEB/A-034 |
| | **Stufe A (Trockene Mischung)** | | |
| 1 | Lactose-Monohydrat | 141,55 | 141,55 |
| 2 | Cellulose, mikrokristallin Ph. Eur. | 50,00 | 50,00 |
| 3 | Crospovidone Ph. Eur. (Polyplasdone^{®}XL 10) | 6,00 | 6,00 |

| **Stufe B (Feste Dispersion, Herstellung und Granulierung)** | | | |
|---|---|---|---|
| 1 | Nebivolol-Hydrochlorid | 5,45 | 5,45 |
| 2 | Hydroxypropylcellulose Ph. Eur. (Klucel^{®} LF) | 5,00 | -- |
| 3 | Hydroxypropylmethylcellulose 5 Cps (Methocel E5) | -- | 5,00 |
| 4 | Wasser, ger. Ph. Eur. | q.s. | q.s. |

| **Stufe C (Mischen u. Gleitmittel)** | | | |
|---|---|---|---|
| 1 | Magnesiumstearat Ph. Eur. | 1,00 | 1,00 |
| 2 | Crospovidone Ph. Eur. (Polyplasdone^{®}XL 10) | 6,00 | 6,00 |
| | Gesamt | 215,00 | 215,00 |

### Verfahren

Lactosemonohydrat, mikrokristalline Cellulose und Crospovidone werden durch ein 50-mesh-Sieb gesiebt und in einen RMG gegeben und 5 Minuten lang gemischt.

Getrennt hiervon werden Hydroxypropylcellulose oder Hydroxypropylmethylcellulose in gereinigtem Wasser gelöst und dann Nebivolol-Hydrochlorid darin dispergiert. Dann wird mit der Mischung von Stufe 1 granuliert.

Anschließend wird die nasse Masse durch ein 10-mesh-Sieb gesiebt und in einem Schnelltrockner bei 60°C getrocknet, um LOD unter 2% zu erhalten.

Die durch 20 mesh gesiebten getrockneten Körnchen werden in einen Doppelschneckenmischer gegeben und 1 Minute lang gemischt.

Magnesiumstearat und Crospovidone werden durch 40-mesh-Sieb gesiebt und nach Stufe 3.4 zugegeben und 5 Minuten lang gemischt.

Die mit Schmiermittel versehene Mischung wird zu runden Tabletten in einem Tablettierwerkzeug gepresst.

## Patentansprüche

1. Pharmazeutische Zusammensetzung enthaltend als Wirkstoff Nebivolol oder ein pharmazeutisch annehmbares Salz davon und ein pharmazeutisch annehmbares Polymerträgermaterial umfassend oder bestehend aus Polyvinylpyrrolidon-co-vinylacetat, wobei der Wirkstoff in mikronisierter Form in der Polymerträgermatrix vorliegt und im Wesentlichen frei von Polysorbat 80 als Netzmitteln ist, die erhältlich ist dadurch, dass eine Dispersion aus einer wässrigen Lösung des Polymerträgers und des Wirkstoffs gebildet wird, getrennt davon ein oder mehrere Füllstoffe mit übrigen Bestandteilen vermischt werden, die wässrige Dispersion den Füllstoffen unter Rühren zugefügt wird und anschließend die Masse nass granuliert wird.

2. Pharmazeutische Zusammensetzung nach Anspruch 1, **dadurch gekennzeichnet, dass** die Wirkstoffteilchen in mikronisierter Form eine spezifische Oberfläche von mehr als 1,8 m²/g haben.

3. Pharmazeutische Zusammensetzung nach Anspruch 2, **dadurch gekennzeichnet, dass** die mikronisierten Wirkstoffteilchen eine spezifische Oberfläche von mehr als 2,8 m²/g haben.

4. Pharmazeutische Zusammensetzung nach Anspruch 2, **dadurch gekennzeichnet, dass** die mikronisierten Wirkstoffteilchen eine spezifische Oberfläche von mehr als 3,0 m²/g haben.

5. Pharmazeutische Zusammensetzung nach einem der vorhergehenden Ansprüche, **dadurch gekennzeichnet, dass** die Wirkstoffteilchen einen volumenmittleren Teilchendurchmesser von 1 bis 20 µm haben.

6. Pharmazeutische Zusammensetzung nach einem der vorhergehenden Ansprüche, **dadurch gekennzeichnet, dass** zusätzlich zu dem Wirkstoff, der Polymerträgermatrix und den Füllstoffen, Schmiermittel, Sprengmittel und/oder Aromastoffe als übrige Bestandteile enthalten sind.

7. Pharmazeutische Zusammensetzung nach einem der vorhergehenden Ansprüche, **dadurch gekennzeichnet, dass** als Füllstoffe Lactose, mikrokristalline Cellulose und/oder Calciumhydrogenphosphat enthalten sind.

8. Pharmazeutische Zusammensetzung nach einem der vorhergehenden Ansprüche, **dadurch gekennzeichnet, dass** zusätzlich chemisch vernetzte Polymere oder Copolymere enthalten sind.

9. Pharmazeutische Zusammensetzung nach einem der vorhergehenden Ansprüche, **dadurch gekennzeichnet, dass** sie in Form von Tabletten vorliegen.

10. Pharmazeutische Zusammensetzung nach einem der vorhergehenden Ansprüche, **dadurch gekennzeichnet, dass** Wirkstoff und Trägerpolymermatrix in einem Verhältnis von 1:0,8 bis 1:10 enthalten sind.

11. Pharmazeutische Zusammensetzung nach einem der vorhergehenden Ansprüche, **dadurch gekennzeichnet, dass** Wirkstoff und Trägerpolymermatrix in einem Verhältnis von 1:1 bis 1:10 enthalten sind.

12. Pharmazeutische Zusammensetzung nach einem Anspruch 10 oder 11, **dadurch gekennzeichnet, dass** Wirkstoff und Trägerpolymermatrix in einem Verhältnis von 1:1 bis 1:6 vorhanden sind.

13. Pharmazeutische Zusammensetzung nach einem der vorhergehenden Ansprüche, **dadurch gekennzeichnet, dass** die Zusammensetzung in Form von Tabletten vorliegt und solche Auflösungseigenschaften hat, dass nach 60 Minuten unter Magensaftbedingungen mindestens 80 % des Wirkstoffs gelöst sind.

14. Pharmazeutische Zusammensetzung nach einem der vorhergehenden Ansprüche, **dadurch gekennzeichnet, dass** sie in Form von Tabletten vorliegt mit Auflösungseigenschaften, so dass nach 60 Minuten unter Magensaftbedingungen mindestens 90 Gew.-% des Wirkstoffs aufgelöst sind.

15. Verfahren zur Herstellung einer pharmazeutischen Zusammensetzung, die als Wirkstoff mikronisiertes Nebivolol oder ein pharmazeutische annehmbares Salz davon in einer Polymerträgermatrix umfassend oder bestehend aus Polyvinylpyrrolidon-co-vinylacetat enthält, bei dem eine Dispersion aus einer wässrigen Lösung des Polymerträgers umfassend oder bestehend aus Polyvinylpyrrolidon-co-vinylacetat und des mikronisierten Wirkstoffs gebildet wird, getrennt davon ein oder mehrere Füllstoffe mit den übrigen Bestandteilen vermischt werden, die wässrige Dispersion den Füllstoffen unter Rühren zugefügt wird und anschließend die Masse nass granuliert wird und aus den Körnchen Tabletten gepresst werden.

16. Verwendung von Nebivolol zur Herstellung einer pharmazeutischen Zusammensetzung gemäß einem der Ansprüche 1 bis 14 zur Behandlung von Hypertonie.

## Claims

1. Pharmaceutical composition containing as active ingredient Nebivolol or a pharmaceutically acceptable salt thereof, comprising or consisting of polyvinylpyrrolidone-co-vinylacetate, wherein the active ingredient is present in the polymer carrier matrix in micronized form and is substantially free of Polysorbate 80 as a wetting agent, obtainable by forming a dispersion from an aqueous solution of the polymer and the active ingredient, therefrom separated admixing one or more fillers with remaining components, adding under agitation the aqueous dispersion to the fillers and subsequently granulating the mass under wet conditions.

2. Pharmaceutical composition according to claim 1, **characterized in that** the micronized active ingredient particles in micronized form have a specific surface of more than 1.8 m²/g.

3. Pharmaceutical composition according to claim 2, **characterized in that** the micronized active ingredient particles have a specific surface of more than 2.8 m²/g.

4. Pharmaceutical composition according to claim 2, **characterized in that** the micronized active ingredient particles have a specific surface of more than 3.0 m²/g.

5. Pharmaceutical composition according to one of the preceding claims, **characterized in that** the active ingredient particles have a mean volume particle diameter from 1 to 20 µm.

6. Pharmaceutical composition according to one of the preceding claims, **characterized in that** in addition to the active ingredient, the polymer carrier matrix and the fillers, as remaining components lubricants, disintegrants and / or flavoring agents are contained.

7. Pharmaceutical composition according to one of the preceding claims, **characterized in that** as fillers lactose, microcrystalline cellulose and / or Calcium hydrogenphosphate are contained.

8. Pharmaceutical composition according to one of the preceding claims, **characterized in that** in addition chemical cross-linked polymers or copolymers are contained.

9. Pharmaceutical composition according to one of the preceding claims, **characterized in that** they are present in form of tablets.

10. Pharmaceutical composition according to one of the preceding claims, **characterized in that** active ingredient and polymer carrier matrix are contained in a ratio from 1:0.8 to 1:10.

11. Pharmaceutical composition according to one of the preceding claims, **characterized in that** active ingredient and polymer carrier matrix are contained in a ratio from 1:1 to 1:10.

12. Pharmaceutical composition according to one of the preceding claims, **characterized in that** active ingredient and polymer carrier matrix are containing in a ratio from 1:1 to 1:6.

13. Pharmaceutical composition according to one of the preceding claims, **characterized in that** the composition is present in form of tablets and has such dissolution properties, that after 60 minutes under gastric juice conditions at least 80 % of the active ingredient is dissolved.

14. Pharmaceutical composition according to one of the preceding claims, **characterized in that** the composition is present in form of tablets and has such dissolution properties, that after 60 minutes under gastric juice conditions at least 90 % of the active ingredient is dissolved.

15. Process of producing a pharmaceutical composition, which contains as active ingredient micronized Nebivolol or a pharmaceutical acceptable salt thereof in a polymer carrier matrix comprising or consisting of polyvinylpyrrolidone-co-vinylacetate, wherein a dispersion of an aqueous solution of the polymer carrier comprising or consisting of polyvinylpyrrolidone-co-vinylacetate and the micronized active ingredient is formed, therefrom separated one or more fillers are admixed with the remaining components, the aqueous dispersion is added under agitation to the fillers and subsequently the mass is being granulated under wet conditions and tablets are compressed from the granules.

16. Use of Nebivolol in the manufacture of a pharmaceutical composition according to one of claims 1 to 14 in the treatment of hypertension.

## Revendications

1. Composition pharmaceutique contenant en tant qu'agent du Nébivolol ou un sel pharmaceutiquement acceptable de cet agent et un matériau porteur de polymère pharmaceutiquement acceptable comportant ou consistent en polyvinylepyrrolidone-co-acétate de vinyle, l'agent existant en forme micronisée dans la matrice porteuse de polymère et étant sensiblement libre de polysorbate 80 en tant qu'agents mouillants qui est procurable par formation d'une dispersion à partir d'une solution aqueuse du porteur de polymère et de l'agent, mélange séparé d'un ou plusieurs agents de charge avec des autres composants, addition de la dispersion aqueuse aux agents de charge par agitation et ensuite granulation humide de la masse.

2. Composition pharmaceutique selon la revendication 1, **caractérisé en ce que** les particules d'agent en forme micronisée ont une surface spécifique de plus de 1,8 m²/g.

3. Composition pharmaceutique selon la revendication 2, **caractérisé en ce que** les particules d'agent micronisées ont une surface spécifique de plus de 2,8 m²/g.

4. Composition pharmaceutique selon la revendication 2, **caractérisé en ce que** les particules d'agent micronisées ont une surface spécifique de plus de 3,0 m²/g.

5. Composition pharmaceutique selon l'une quelconque des revendications précédentes, **caractérisé en ce que** les particules d'agent ont un diamètre moyen de particule en volume de 1 à 20 µm.

6. Composition pharmaceutique selon l'une quelconque des revendications précédentes, **caractérisé en ce qu'**en outre l'agent, la matrice porteuse de polymère et les agents de charge, des matériaux lubrifiants, des désintégrants et/ou des substances aromatisantes sont contenus dans la composition en tant que d'autres composants.

7. Composition pharmaceutique selon l'une quelconque des revendications précédentes, **caractérisé en ce que** du lactose, de la cellulose microcristalline et/ou du hydrogéno-orthophosphate de calcium sont contenus en tant que d'agents de charge.

8. Composition pharmaceutique selon l'une quelconque des revendications précédentes, **caractérisé en ce qu'**en outre des polymères ou copolymères réticulés chimiquement sont contenus dans la composition.

9. Composition pharmaceutique selon l'une quelconque des revendications précédentes, **caractérisé en ce qu'**elles existent sous forme de comprimés.

10. Composition pharmaceutique selon l'une quelconque des revendications précédentes, **caractérisé en ce que** l'agent et la matrice porteuse de polymère sont contenus en un rapport 1:0,8 jusqu'au 1:10.

11. Composition pharmaceutique selon l'une quelconque des revendications précédentes, **caractérisé en ce que** l'agent et la matrice porteuse de polymère sont contenus en un rapport 1:1 jusqu'au 1:10.

12. Composition pharmaceutique selon la revendication 10 ou 11, **caractérisé en ce que** l'agent et la matrice porteuse de polymère sont contenus en un rapport 1:1 jusqu'au 1:6.

13. Composition pharmaceutique selon l'une quelconque des revendications précédentes, **caractérisé en ce que** la composition existe sous forme de comprimés et possède des propriétés de dissolution de manière qu'après 60 minutes sous des conditions de suc gastrique au moins 80 % de l'agent sont dissolus.

14. Composition pharmaceutique selon l'une quelconque des revendications précédentes, **caractérisé en ce qu'**elle existe sous forme de comprimés avec des propriétés de dissolution de manière qu'après 60 minutes sous des conditions de suc gastrique au moins 90 pour cent en poids de l'agent sont dissolus.

15. Procédé pour la fabrication d'une composition pharmaceutique contenant en tant qu'agent du Nébivolol micronisé ou un sel pharmaceutiquement acceptable de cet agent dans une matrice porteuse de polymère comportant ou consistent en polyvinylepyrrolidone-co-acétate de vinyle, une dispersion à partir d'une solution aqueuse du porteur de polymère comportant ou consistent en polyvinylepyrrolidone-co-acétate de vinyle et de l'agent micronisé étant formée, un ou plusieurs agents de charge étant mélangés avec des autres composants séparément de cette dispersion, la dispersion aqueuse étant ajoutée aux agents de charge par agitation et ensuite la masse étant granulée d'une manière humide et des comprimés étant agglomérés par compression à partir des granules.

16. Utilisation de Nébivolol pour la fabrication d'une composition pharmaceutique selon l'une quelconque des revendications 1 à 14 pour le traitement de l'hypertonie.
